# EUROPEAN PATENT SPECIFICATION

(11) **EP 1 586 328 B1**
(45) Date of publication and mention of the grant of the patent: **20.02.2013**
(21) Application number: 03768290.3
(22) Date of filing: 26.12.2003
(51) Int. Cl.: A61K 38/48, A61P 7/02, A61P 9/00, A61P 9/10, A61P 43/00

(54) **AGENT FOR IMPROVING LIFE EXPECTANCY IN THERAPY OF MALIGNANT TUMOR**
WIRKSTOFF ZUR ERHÖHUNG DER LEBENSERWARTUNG BEI DER THERAPIE BÖSARTIGER TUMORE
AGENT D'AMELIORATION D'ESPERANCE DE VIE DANS LE TRAITEMENT DE TUMEUR MALIGNE

(30) Priority: 27.12.2002 JP 2002379409; 07.05.2003 JP 2003129208
(43) Date of publication of application: 19.10.2005
(73) Proprietor: The Chemo-Sero-Therapeutic Research Institute, Kumamoto-shi Kumamoto 860-8568 (JP)
(72) Inventor: OKAJIMA, Kenji, Kumamoto-shi, Kumamoto 862-0926 (JP); MATSUO, Fujio Jur.Found. The Chemo-Sero-Th. Res., Kikuchi-shi Kumamoto-ken 869-1298 (JP); SUTOH, Hiroyuki Jur.Found. The Chemo-Sero-Th. Res., Kikuchi-shi Kumamoto-ken 869-1298 (JP); OGATA, Yoichi JUR. FOUND. THE CHEMO-SERO-THERA., Kumamoto-shi, Kumamoto 860-8568 (JP); NAKAGAKI, Tomohiro Jur.Found. The Chemo-Sero-Ther., Kikuchi-shi Kumamoto-ken 869-1298 (JP)
(74) Representative: Vossius & Partner
(86) International application number: PCT/JP2003/016858
(87) International publication number: WO 2004/060395

(56) References cited:
- EP-A- 0 191 606
- JOYCE DAVID ET AL: "IMPROVEMENT OF DISSEMINATED INTRAVASCULAR COAGULATION IN SEVERE SEPSIS PATIENTS TREATED WITH RECOMBINANT HUMAN ACTIVATED PROTEIN C" and "DISSEMINATED INTRAVASCULAR COAGULATION IN SEVERE SEPSIS PATIENTS TREATED WITH RECOMBINANT HUMAN ACTIVATED PROTEIN C, A RETROSPECTIVE SUBGROUP ANALYSIS" BLOOD, AMERICAN SOCIETY OF HEMATOLOGY, US, vol. 98, no. 11 PART 1, 11 December 2001 (2001-12-11), page 445A (ABSTRACT1865 AND ABSTRACT1866), XP008075901 ISSN: 0006-4971
- WADA H ET AL: "THROMBOMODULIN INHIBITS TISSUE FACTOR-INDUCED THROMBIN GENERATION IN PLASMA OF PATIENTS WITH DISSEMINATED INTRAVASCULAR COAGULATION" BLOOD, AMERICAN SOCIETY OF HEMATOLOGY, US, vol. 98, no. 11, PART 2, 1 January 2001 (2001-01-01), page 78B,ABSTR.NO.#3937, XP003021736 ISSN: 0006-4971
- GOUIN-THIBAULT I ET AL: "The thrombophilic state in cancer patients" ACTA HAEMATOLOGICA (BASEL), vol. 106, no. 1-2, September 2001 (2001-09), pages 33-42, XP009118954 ISSN: 0001-5792
- AMIRKHOSRAVI ALI ET AL: "Activated protein C inhibits tumor cell-induced coagulation activation in vivo and suppresses experimental lung metastasis." BLOOD, vol. 102, no. 11, 16 November 2003 (2003-11-16), page 14a, XP009118980 & 45TH ANNUAL MEETING OF THE AMERICAN SOCIETY OF HEMATOLOGY; SAN DIEGO, CA, USA; DECEMBER 06-09, 2003 ISSN: 0006-4971
- LORETO M. F. ET AL.: 'Coagulation and cancer: Implications for diagnosis and management' PATHOLOGY ONCOLOGY RESEARCH vol. 6, no. 4, 2000, pages 301 - 312, XP002904657
- MITATE YASUO ET AL.: 'Kessensho no rinsho-ekigaku to byotai DIC-byotai no tayosei' MEDICINA vol. 37, no. 5, 10 May 2000, pages 735 - 737, XP002904658
- IKEDA YASUO: 'Atarashii kokessen'yaku kakuron1) kogyokoyaku (2) kasseika protein C' IRYO JOURNAL vol. 35, no. 4, 01 April 1999, pages 1061 - 1066, XP002904659
- KATSUURA YASUHIRO: 'Kokessen'yaku no genjo to shin'yaku kaihatsu kasseika protein C no kokessen sayo' FOLIA PHARMACOLOGICA JAPONICA vol. 116, no. 5, 01 November 2000, pages 290 - 297, XP002904660

## Description

### TECHNICAL FIELD OF THE INVENTION

The present invention belongs to the field of a medical drug. Specifically, the present invention relates to a novel use of a plasma protein. More specifically, the present invention relates to a medicament for use in improving prognostic survival in chemotherapy of malignant tumor in hematopoietic organs, wherein said malignant tumor is accompanied by DIC, and wherein said medicament comprises as a main active ingredient Activated Protein C (hereinafter also referred to as "APC"). The medicament may increase a survival rate of patients suffering from malignant tumor.

### BACKGROUND OF THE INVENTION

Malignant tumor ranks first in the causes of death in Japanese and has a continued tendency of increase in number nowadays. In therapy of various malignant tumors ranging from tumors in the hematopoietic system such as leukemia to solid cancers, improvement in prognosis for prolonging life-span is desired.

A number of chemotherapeutics have been used for therapy of malignant tumor. Many of these chemotherapeutics are so-called cytotoxic agents that kill cells by directly acting to and damaging DNAs and RNAs. Since these cytotoxic agents exert their cytotoxic activity not only to target tumor cells but also to normal cells, a dose has been designed on the assumption that adverse side effects would be somehow inevitable. Namely, a dosage regimen for chemotherapeutics that is most distinct from other medicaments is that ordinary therapy is made with as high dose as possible tolerable to patients (maximum tolerated dose; MTD) since with higher dose chemotherapeutics will provide higher clinical efficacy.

For ordinary medicaments, a minimum dose for providing therapeutic efficacy would scarcely induce adverse side effects from clinical point of view and hence their therapeutic range is broad. On the contrary, in case of chemotherapeutics, clinical efficacy, typically of prolonging life-span, would be provided only when a dose is used that exceeds one accompanied by adverse side effects such as thrombosis/DIC, gastrointestinal toxicity, bone marrow suppression, alopecia and the like. There are also many cases where patients suffer from adverse side effects but with no substantial clinical efficacy. Besides, under these circumstances it is not rare that fatal side effects occur.

In order to solve these problems, novel chemotherapeutics have been developed, or a dosage regimen of chemotherapeutics has been devised in various manners, or a combined therapy has been performed so as to decrease adverse side effects. However, a clinically useful method for treatment has not yet been established that may improve prognostic survival in patients suffering from malignant tumor (see "Novel diagnosis and therapy for cancer", ed. by Hiroo Imura).

It is known that patients suffering from malignant tumor tend to onset thrombosis since many malignant tumor cells have procoagulant properties that are enhanced by administration of chemotherapeutics (see Am. J. Hematol., vol. 72, p.43-52, 2003). There are reports that 60% of patients suffering from malignant tumor are in conditions of hypercoagulation (see Ann. Clin. Lab. Sci., vol. 24, p.1-5, 1994) and that 15% of the patients develop thrombotic symptoms (see Semin. Thromb. Hemost., vol. 18, p.373-379, 1992). From this reason, chemotherapeutics have been combined with anti-coagulant therapy using heparin or warfarin with expectation of high efficacy in therapy of malignant tumor (see Haemostasis, vol. 16, p.300-320, 1986).

In fact, it is demonstrated that heparin, a typical anti-coagulant, has statistically significant life-prolonging effect when added to the conventional therapy for malignant tumor in various retrospective (see Ann. Intern. Med., vol. 96, p.561-565, 1982; Int. J. Colorectal. Dis., vol. 8, p.111-115, 1993; Surgery, vol. 93, p.433-438, 1983) or prospective (see Cancer, vol. 74, p.38-45, 1994) clinical research, suggesting usefulness of heparin in patients suffering from malignant tumor (see Thromb. Haemost., vol. 80, p.10-23, 1998). However, the effect of heparin is still insufficient in view of prognostic survival.

Among typical thromboses caused by malignant tumor is DIC (disseminated intravascular coagulation). DIC is a syndrome where the coagulation system is continually activated so far as to hypercoagulation due to basal diseases such as malignant tumor and infectious diseases to thereby induce frequent occurrence of thrombus mainly in the microvasculature, leading to ischemic organopathy. DIC is a disease with an extremely high lethality. In DIC, hemorrhage is also observed, in addition to thrombosis, which is as a consequence of consumption coagulopathy (i.e. coagulopathy due to decrease in coagulation factors and platelets necessary for hemostasis as a result of consumption) and secondary hyperfibrinolysis (i.e. rise in degradation of fibrin thrombus) caused by hypercoagulation. As such, DIC is characterized by that in spite of its extreme tendency towards thrombosis it conversely exhibits frequent hemorrhage, making DIC therapy difficult.

Mechanism for onset of DIC may differ depending on basal diseases. In case that the basal disease is malignant tumor, cancer cells or leukemic cells may often express tissue factors as a trigger of coagulation reaction. In particular, when these cells were destroyed by the use of chemotherapeutics, release of the tissue factors from these cells may drastically be induced to thereby invoke hypercoagulation. In case of DIC caused by malignant tumor, prognostic survival is extremely poor and hence its improvement has been desired. On the other hand, it is supposed that inflammatory cytokines are deeply involved in DIC caused by sepsis and thus usefulness of anti-coagulants with anti-inflammatory activity is suggested.

Among anti-coagulants that are widely used internationally for DIC therapy are heparins (not fractionated heparin, low molecular weight heparin, heparan sulfate). Although heparins possess a high anti-coagulating activity, they have a defect that they tend to induce hemorrhage. In Japan, synthetic protease inhibitors (FOY; FUTHAN) and anti-thrombin III (ATIII) have also been used as DIC medicaments. However, while synthetic protease inhibitors are not inclined to induce hemorrhage, their anti-coagulating activity is lower than that of heparins. ATIII has the same defect as heparins since it is ordinarily used concurrently with heparins.

These DIC medicaments have been indicated to ameliorate the condition of hypercoagulation through their anti-coagulating activity. However, no medicaments are known that are proved to notably improve prognostic survival of DIC patients, which is however an ultimate object, and thus development of medicaments that may improve prognostic survival of DIC patients is desired. Clinical test for approved DIC medicaments assesses their clinical efficacy with DIC Score determined by DIC research team in the Welfare Ministry (the Welfare Ministry, Research team for specified diseases, coagulopathy, Report in 1992, p.37-41, 1988) but does not assess prognostic survival.

On the other hand, contrary to the usefulness of anti-coagulants, there are reports that an anti-tumor effect was obtained by administering coagulation factors (Activated Factor IX; Tissue Factor that triggers coagulation reaction (Factor III)) alone or in combination with cytokines to induce activated conditions of coagulation (see Japanese Patent Publication No. 10-501813) and that an excellent anti-tumor effect was obtained by administering a factor that inhibits Protein C anti-coagulation system, i.e. a major control system of coagulation in the living body, alone or in combination with anti-tumor agents (see U.S. Patent No.5147638).

APC circulates within the blood vessel in the form of its precursor Protein C (PC). Once the coagulation system is triggered and thrombin is formed, thrombin binds to the membrane protein on the vascular endothelial cells called thrombomodulin (TM) to transform PC into APC having a serine protease activity through activation. APC on phospholipids of the cellular membrane selectively acts on activated Factor V and activated Factor VIII of the blood coagulation system for restricted degradation and inactivation of these factors to thereby display a potent anti-coagulation activity (Biochemistry, vol.16, p.5824-5831, 1977; J. Biol. Chem., vol. 258, p.1914-1920, 1982). This anti-coagulation activity by APC may be enhanced in the presence of cofactor Protein S (PS). The coagulation control system in which PC, TM, and PS are involved is called Protein C anti-coagulation system.

On the other hand, APC is thought to be involved in promotion of fibrinolysis by neutralizing tissue plasminogen activator inhibitor (PAI) derived from the vascular endothelial cells or platelets (Proc. Natl. Acad. Sci. USA, vol. 82, p.1121-1125, 1985; J. Biol. Chem., vol. 276, p.15567-15570, 2001) or by inhibiting activation of anti-fiblinolytic factor TAFI (Thrombin Activatable Fibrinolysis Inhibitor) (Blood, vol. 88, p.2093-2100, 1996).

Besides, it is suggested that APC has an anti-inflammatory activity since APC has proved to be effective in septic model (J. Clin. Invest., vol.79, p.918-925, 1987) and to inhibit cytokine production in leucocytes (Am. J. Physiol., p.L197-L202, 1997). Clinically, in a large-scale clinical test performed by Eli Lilly for patients suffering from severe sepsis, it was demonstrated that administration of a recombinant APC preparation significantly reduced lethality of patients and a level of an inflammatory cytokine IL-6 was significantly reduced 1 day after administration of the recombinant APC (N. Engl. J. Med., vol. 344, p699-709, 2001). As a result of retrospective analysis of the above clinical test, rAPC administration lowered a relative risk (RR) of lethality by 19.4% when assessed for the whole patients suffering from severe sepsis (N=1690) whereas it lowered RR of lethality by 42% for DIC patients (N=221) evidently caused by sepsis, indicating that treatment of DIC caused by sepsis with APC exceedingly improved lethality (Blood, vol. 98, 445, 2001). However, for DIC caused by non-inflammatory, basal diseases other than sepsis, it was utterly unknown whether APC could improve prognostic survival.

Document EP-A-0 191 606 discloses a specific double-stranded recombinant DNA vector that encodes a polypeptide with human protein C activity.
M. F. Loretto et al., Pathology Oncology Research, 2000, vol. 6, no. 4, 301 - 312 discloses coagulation and cancer: implications for diagnosis and management.
D. Joyce, et al., American Society of Hematology, 2001, vol. 98, no. 11, part 1, 445a, abstract 1865 discloses improvement of disseminated intravascular coagulation in severe sepsis patients treated with recombinant human Activated Protein C.
D. Joyce, et al., American Society of Hematology, 2001, vol. 98, no. 11, part 1, 445a, abstract 1866 discloses disseminated intravascular coagulation in severe sepsis patients treated with recombinant human Activated Protein C.

### DISCLOSURE OF THE INVENTION

### Technical Problem to be Solved by the Invention

As mentioned above, although therapy for malignant tumor, typically chemotherapeutics, has nowadays made rapid progress, the above problems have not yet well overcome and thus further advancement of therapeutic efficacy by chemotherapeutics against malignant tumor is desired.

### Means for Solving the Problems

Under the circumstances, the present inventors have earnestly investigated so as to find out medicaments that improve prognostic survival in therapy of malignant tumor and accessory medicaments for chemotherapy that mitigate the problems of chemotherapeutics. As a result, the present inventors have surprisingly found that: (1) the anti-coagulant APC, which no researchers have ever attempted, had an effect improving prognostic survival of patients suffering from malignant tumor who previously received administration of chemotherapeutics; (2) said effect was significantly higher than that of heparins, a life-prolonging effect of which has already been suggested for patients suffering from malignant tumor; and (3) in particular, APC significantly improved survival rate of patients of DIC caused by malignant tumor. Based on these findings, the present inventors have completed the invention of the instant application.

Namely, the present invention relates to medicaments for use in improving prognostic survival in chemotherapy of malignant tumor in hematopoietic organs, accompanied by DIC, comprising as a main active ingredient Activated Protein C. In the following the term "malignant tumor" is defined as "malignant tumor in hematopoietic organs, wherein said malignant tumor is accompanied by DIC" when the present invention is referred to. In particular, the medicaments for improving prognostic survival in therapy of malignant tumor of the present invention may efficiently reduce adverse side effects induced by chemotherapeutics, especially thrombosis, by combining the medicaments with chemotherapeutics while chemotherapy of malignant tumor. In such a case, the medicaments for improving prognostic survival of the present invention may be used as accessory medicaments for chemotherapy against malignant tumor based on the activity of APC to reduce adverse side effects of chemotherapeutics.

These findings are based on the analysis of results of DIC therapy with APC or heparins for patients of DIC caused by malignant tumor as the basal disease.

### More Efficacious Effects than Prior Art.

By the findings obtained by the present invention, prognostic survival in therapy of malignant tumor may be improved and, when chemotherapeutics are administered against malignant tumor, accessory medicaments are provided that may reduce the conventional adverse side effects of chemotherapeutics and enhance the activity of chemotherapeutics to kill tumor cells to thereby exceedingly improve prognostic survival of patients. We are convinced that the present invention may exert such excellent effects and would greatly contribute to the art field relevant to the present invention.

### BRIEF DESCRIPTION OF DRAWINGS

Fig. 1 shows results of survival time analysis in DIC patients as a whole administered with APC composition or heparin as a control.
Fig. 2 shows results of survival time analysis in patients of DIC caused by malignant tumor in hematopoietic organs administered with APC composition or heparin as a control.
Fig. 3 shows survival time analysis in patients suffering from malignant tumor administered with APC or heparin as a control where chemotherapeutics were not concurrently used.
Fig. 4 shows survival time analysis in patients suffering from malignant tumor administered with APC or heparin as a control where chemotherapeutics were concurrently used.

### BEST MODE FOR CARRYING OUT THE INVENTION

The medicaments for use in improving prognostic survival in chemotherapy of malignant tumor and the accessory medicaments for chemotherapy against malignant tumor in accordance with the present invention are characterized by that they comprise as a main active ingredient APC.

In case that the medicaments for use in improving prognostic survival of the present invention are used as accessory medicaments for chemotherapy against malignant tumor, chemotherapeutics against malignant tumor may include, but not limited to, commonly used chemotherapeutics including, for instance, plant alkaloids such as vincristine sulfate, alkylating agents such as cyclophosphamide, anti-cancer antibiotics such as daunorubicin sulfate, antimetabolites such as cytarabine, and platinum preparations such as cisplatin. Also, concurrent use of any combination of these chemotherapeutics may be encompassed by the technical idea of the present invention. The accessory medicaments for chemotherapy of the present invention may be administered in any mode, for instance, prior to or simultaneously with initiation of the administration of chemotherapeutics in preferable embodiments.

APC, as an active ingredient of the medicaments for use in improving prognostic survival in chemotherapy of malignant tumor and the accessory medicaments for chemotherapy against malignant tumor in accordance with the present invention, may be prepared by any known techniques. It may be prepared, for instance, by activating PC isolated from human blood or obtained by the genetic recombination technique, by isolating APC from human blood, or by directly preparing APC by the genetic recombination technique. Activation of PC into APC may also be performed by any known techniques, for instance, by activation with thrombin isolated from human or bovine blood, or by activation with a recombinant thrombin.

APC may be prepared from blood as described below. For instance, APC may be prepared by the process disclosed in Japanese Patent No. 3043558; or by purifying PC from human plasma by affinity chromatography with anti-PC antibody, activating the purified PC with human thrombin, and then purifying the resultant APC by cation chromatography (Blood, vol. 63, p.115-121, 1984); by the process according to Kisiel, i.e. by purifying PC from human plasma by the steps of absorption with Ba citrate and elution, ammonium sulfate fractionation, DEAE-Sephadex column chromatography, dextran sulfate agarose chromatography and polyacrylamide gel electrophoresis, and activating the purified PC (J. Clin. Invest., vol. 64, p.761-769, 1979); or by purifying PC by affinity chromatography with anti-PC antibody using as a starting material commercially available Factor IX complex preparations containing PC, and activating the purified PC (J. Clin. Invest., vol. 79, p.918-925, 1987).

APC may also be prepared by using the genetic recombination techniques, for instance, in accordance with the processes disclosed in Japanese patent publication No. 205487/1986, Japanese patent publication No. 002338/1989 or Japanese patent publication No. 085084/1989.

Once APC has been prepared as described above, it may be stored by lyophilization together with a suitable stabilizing agent so as to keep the activity of APC at maximum. In accordance with the present invention, APC as an active ingredient may be combined with appropriate known fillers by the methods known in the art to prepare "the medicaments for improving prognostic survival in chemotherapy of malignant tumor" and "the accessory medicaments for chemotherapy against malignant tumor" of the present invention. A dose of the medicaments of the present invention comprising APC as an active ingredient may vary depending on various factors such as age and conditions of patients but preferably in a range of from 50 to 3000 units/kg/day, for instance, which may continually be administered for 3 to 6 days. One unit of APC activity is defined as an amount of activity contained in 1 ml of a sample that may twice prolong Activated Partial Thromboplastin Time (APTT) when equivalent amounts of normal plasma and a sample to be measured containing APC are mixed together.

The present invention is explained in more detail by means of the following Examples but should not be construed to be limited thereto.

APC derived from blood to be used in these Examples has been proved for its safety by a single-dose intravenous administration test in mice and dogs, a repetitive intravenous administration test in mice, dogs and infantile dogs, a breeding test in mice, a topical irritation test, a general pharmacological test (effect on the respiratory and circulatory systems using beagles), and a viral inactivation test.

### Example 1

### Efficacy of APC to survival rate in DIC caused by either malignant tumor or infectious diseases.

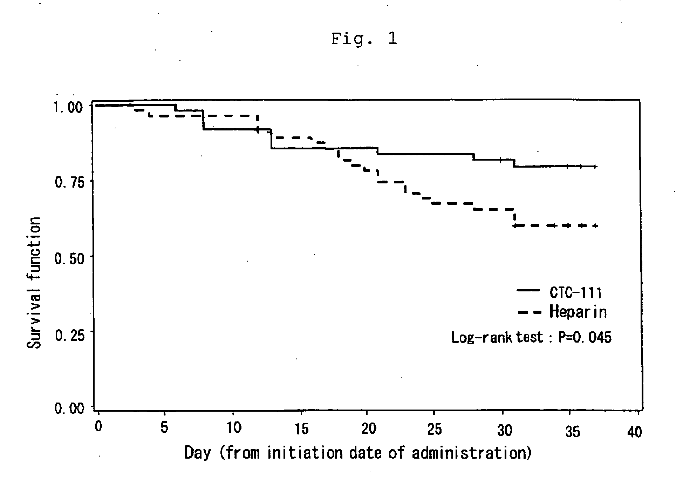

In order to investigate efficacy and safety of APC to DIC caused by leukemia and DIC caused by infectious diseases as a complication of internal diseases, a phase III double-blind test was performed with heparin as a control (Int. J. Hematol., vol. 75, p.540-547, 2002). A dose and a route of administration for each of the preparations were (1) 300 units/kg/day for 6 days for APC (clinical trial code No. CTC111), and (2) 8 units/kg/h for 6 days for heparin. The dose of heparin, 8 units/kg/h, is a standard dose for DIC therapy. A survival rate up till one month of the completion of administration of the preparations was 79.6% (39/49) for APC and 60.0% (33/55) for heparin, indicating 19.6% of a difference between the two groups (see Table 1). As shown in Fig. 1, as a result of survival time analysis, Log-rank test gave P=0.045 (two-side) to indicate superior prognostic survival in APC administration as compared to heparin as a control.

**Table 1: Results of analysis of prognostic survival (DIC patients as a whole)**

| | Survival | Death | Total | Difference of survival rate |
|---|---|---|---|---|
| APC | 39 (79.6%) | 10 (20.4%) | 49 | 19.6% |
| Heparin | 33 (60.0%) | 22 (40.0%) | 55 | |

### Example 2

### Efficacy of APC to survival rate in DIC caused by malignant tumor in hematopoietic organs.

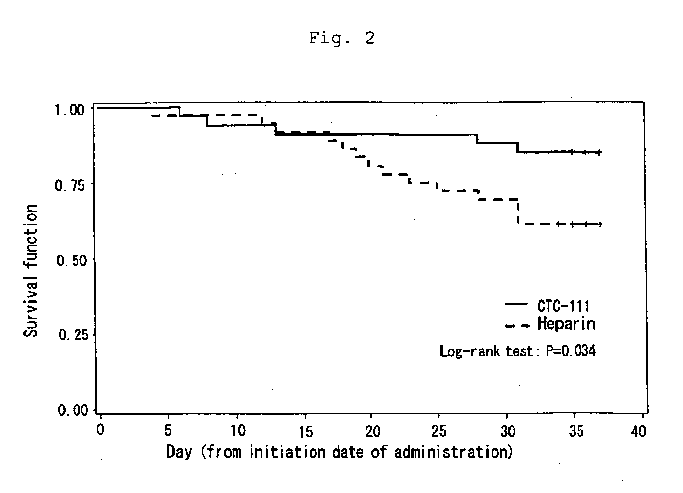

For the results of the above clinical trial, a survival rate after one month of the completion of administration of the preparations was assessed each for the basal diseases of DIC. It was demonstrated that APC administration exceedingly improved a survival rate in DIC caused by malignant tumor in hematopoietic organs. Specifically, a survival rate after one month of the completion of administration of the preparations was 84.8% (28/33) for APC administration whereas it was 61.1% (22/36) for heparin administration (see Table 2). As a result of survival time analysis, Log-rank test gave P=0.034 (two-side) to indicate superior prognostic survival in APC administration as compared to heparin as a control (see Fig. 2).

**Table 2: Results of analysis of prognostic survival (DIC caused by malignant tumor in hematopoietic organs)**

| | Survival | Death | Total | Difference of survival rate |
|---|---|---|---|---|
| APC | 28 (84.8%) | 5 (15.2%) | 33 | 23.7% |
| Heparin | 22 (61.1%) | 14 (38.9%) | 36 | |

### Example 3

### Effect of concurrent use of chemotherapeutics in patients suffering from malignant tumor including solid cancer who received APC administration.

For the results of the above clinical trial, a survival rate after one month of the completion of administration of the preparations was assessed each for the basal diseases of DIC. It was demonstrated that APC administration improved a survival rate in DIC patients suffering from malignant tumor including solid cancer. Specifically, a survival rate up till one month of the completion of administration of the preparations was 77.5% (31/40) for APC administration whereas it was 54.2% (26/48) for heparin administration.

For DIC patients suffering from malignant tumor, the effect of APC was compared with that of heparin as a control with and without concurrent use of chemotherapeutics. It was demonstrated that APC administration could exceedingly lower lethality with concurrent use of chemotherapeutics.

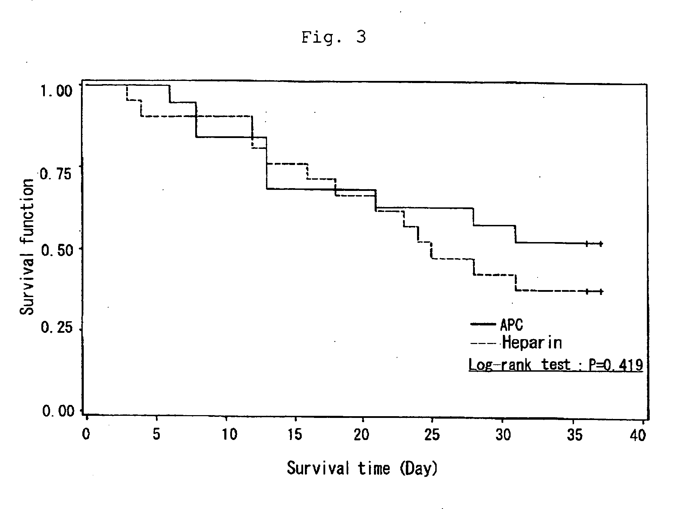

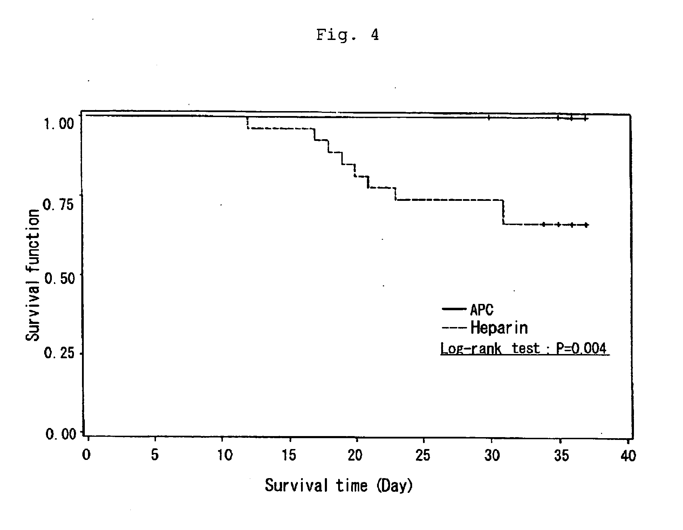

Specifically, without concurrent use of chemotherapeutics, a survival rate up till one month of the completion of administration of the preparations was 52.6% (10/19) for APC administration whereas it was 38.1% (8/21) for heparin administration (see Table 3). As a result of survival time analysis, Log-rank test indicated no significant difference between the two groups (P=0.419 (two-side); see Fig. 3). On the contrary, when chemotherapeutics were concurrently used, a survival rate up till one month of the completion of administration of the preparations was 100% (21/21) for APC administration whereas it was 66.7% (18/27) for heparin administration (see Table 3). As a result of survival time analysis, APC administration improved prognostic survival at a significantly higher rate than heparin administration (Log-rank test; P=0.004 (two-side); see Fig. 4).

**Table 3: Results of analysis of prognostic survival (effect of concurrent use of chemotherapeutics)**

| Without concurrent use of chemotherapeutics | | With concurrent use of chemotherapeutics | |
|---|---|---|---|
| APC | Heparin | APC | Heparin |
| 10/19 (52.6%) | 8/21 (38.1%) | 21/21 (100%) | (18/27 (66.7%) |

### Example 4

### Effect of APC administration on thrombotic therapy in patients suffering from malignant tumor with concurrent use of chemotherapeutics.

It is known that sensitive markers of coagulation/fibrinolysis system, i.e. D dimer, TAT (Thrombin-Antithrombin Complex), FM test and PIC (Plasmin-Plasmin Inhibitor Complex), are useful for diagnosis or assessment of therapeutic efficacy of thrombosis such as deep vein thrombosis (DVT) or pulmonary embolism (PE) (Blood Coagul. Fibrinolysis, vol.11, p.371-377, 2000). These four markers were scored as depicted in Table 4. A total score of these markers was compared between just before administration of the preparations and just after completion of the administration and graded four ("Extreme improvement": improvement in 2 or more in score; "Improvement": improvement in 1 in score; "No change": no change in score; and "Worsening": worsening in 1 or more in score) (this assessment is called comprehensive assessment of FM test etc.) to compare the effect of concurrent use of chemotherapeutics on thrombotic therapy in patients suffering from malignant tumor between APC and heparin administrations. In this respect, "Extreme improvement" was regarded as efficacious whereas "Improvement", "No change" and "Worsening" were regarded as non-efficacious.

**Table 4: Criteria for comprehensive assessment of FM test etc.**

| Markers | Score | | |
|---|---|---|---|
| | 0 | 1 | 2 |
| FM test | - | + | ++, +++ |
| D dimer (ng/mL) | <1000 | 1000-4000 | ≧4000 |
| TAT (ng/mL) | <8 | 8-20 | ≧20 |
| PIC (µg/mL) | <2 | 2-5 | ≧5 |

In the same manner as in Example 3, the effect to prevent thrombus formation was compared between APC and heparin administrations in DIC patients wherein the basal disease is malignant tumor by using the comprehensive assessment of FM test etc. The results are shown in Table 5. Without concurrent use of chemotherapeutics, difference in the efficacy between APC and heparin administrations was 12.7% whereas with concurrent use of chemotherapeutics it was 23.4%. Although statistically significant difference was not shown, APC administration exhibited higher efficacy (anti-thrombotic) than heparin especially when chemotherapeutics were concurrently used.

**Table 5: Effect of concurrent use of chemotherapeutics on comprehensive assessment of FM test etc.**

| Efficacy without concurrent use of chemotherapeutics | | Efficacy with concurrent use of chemotherapeutics | |
|---|---|---|---|
| APC | Heparin | APC | Heparin |
| 10/18 (55.6%) | 9/21 (42.9%) | 13/21 (61.9%) | 10/26 (38.5%) |
| P=0.5278 | | P=0.1968 | |

## Claims

1. A medicament for use in improving prognostic survival in chemotherapy of malignant tumor in hematopoietic organs, wherein said malignant tumor is accompanied by DIC, and wherein said medicament comprises as a main active ingredient Activated Protein C.

2. The medicament according to claim 1, wherein said medicament may reduce thrombus formation induced by chemotherapeutics.

3. Use of Activated Protein C for the preparation of a medicament for improving prognostic survival in chemotherapy of malignant tumor in hematopoietic organs, wherein said malignant tumor is accompanied by DIC, and wherein said medicament comprises Activated Protein C as a main active ingredient.

4. The use according to claim 3, wherein said medicament may reduce thrombus formation induced by chemotherapeutics.

## Patentansprüche

1. Ein Medikament zur Verwendung bei der Verbesserung der Überlebensprognose in der Chemotherapie eines bösartigen Tumors in hämatopoetischen Organen, wobei der bösartige Tumor mit DIC einhergeht, und wobei das Medikament als einen Hauptwirkstoff Aktiviertes Protein C umfasst.

2. Das Medikament gemäß Anspruch 1, wobei das Medikament Chemotherapeutika-induzierte Thrombusbildung verhindern kann.

3. Verwendung von Aktiviertem Protein C für die Herstellung eines Medikaments zur Verbesserung der Überlebensprogrose in der Chemotherapie eines bösartigen Tumors in hämatopoetischen Organen, wobei der bösartige Tumor mit DIC einhergeht, und wobei das Medikament Aktiviertes Protein C als einen Hauptwirkstoff umfasst.

4. Verwendung gemäß Anspruch 3, wobei das Medikament Chemotherapeutika-induzierte Thrombusbildung verhindern kann.

## Revendications

1. Médicament destiné à être utilisé dans l'amélioration de la survie pronostique en chimiothérapie d'une tumeur maligne dans des organes hématopoïétiques, où ladite tumeur maligne est accompagnée par une CIVD, et où ledit médicament comprend comme ingrédient actif principal de la protéine C activée.

2. Médicament selon la revendication 1, où ledit médicament peut réduire la formation de thrombus induite par des substances chimiothérapeutiques.

3. Utilisation de protéine C activée pour la préparation d'un médicament pour améliorer la survie pronostique en chimiothérapie d'une tumeur maligne dans des organes hématopoïétiques, où ladite tumeur maligne est accompagnée par une CIVD, et où ledit médicament comprend de la protéine C activée comme ingrédient actif principal.

4. Utilisation selon la revendication 3, où ledit médicament peut réduire la formation de thrombus induite par des substances chimiothérapeutiques.
